# EUROPÄISCHE PATENTSCHRIFT

(11) **EP 1 153 911 B1**
(45) Veröffentlichungstag und Bekanntmachung des Hinweises auf die Patenterteilung: **23.07.2003**
(21) Anmeldenummer: 01110822.2
(22) Anmeldetag: 04.05.2001
(51) Int. Cl.: C07C 53/134, C07C 29/147, C07C 31/133

(54) **(+)- und (-)-2-Cyclododecylpropanol und (+)- und (-)-2-Cyclododecylpropionsäure sowie deren Herstellung und Verwendung**
(+)- and (-)-2-cyclododecylpropanol and (+)- and (-)-2-cyclododecylpropionic acid and the preparation and use of same
(+)- et (-)-2-cyclododécylpropanol et l'acides de (+) et (-)-2-cyclododécylpropioniques , leurs préparations et utilisations

(30) Priorität: 12.05.2000 DE 10023256
(43) Veröffentlichungstag der Anmeldung: 14.11.2001
(73) Patentinhaber: BASF AKTIENGESELLSCHAFT, 67056 Ludwigshafen (DE)
(72) Erfinder: Ebel, Klaus, Dr., 68623 Lampertheim (DE); Krause, Wolfgang, Dr., 68782 Brühl (DE); Schäfer-Lüderssen, Ulrich, Dr., 67063 Ludwigshafen (DE)

(56) Entgegenhaltungen:
- WO-A-98/56337
- DE-A- 19 725 533
- DE-A- 19 756 171

## Beschreibung

Die vorliegende Erfindung betrifft (+)- und (-)-2-Cyclododecylpropanol und (+)- und (-)-2-Cyclododecylpropionsäure, deren Salze, sowie deren Herstellung und Verwendung.

Das Racemat von 2-Cyclododecylpropanol (auch Hydroxyambran® bzw. Amberwood® genannt) ist ein Riechstoff der Holz- und Ambraklasse, die zunehmend an Bedeutung gewinnt (EP-B278 384). Ein in WO 98/55228 beschriebenes Verfahren zu seiner Herstellung ist die Umsetzung von Cyclododecen in Gegenwart von katalytischen Mengen eines Radikalinitiators mit einem Überschuß an Propionsäure oder an einem Propionsäurederivat und anschließender katalytischer Hydrierung der gebildeten 2-Cyclododecyl-propionsäure bzw. der entsprechenden 2-Cyclododecyl-propionsäure-Derivate.

Sowohl die beiden Enantiomeren von 2-Cyclododecylpropionsäure als auch von 2-Cyclododecylpropanol sind im Stand der Technik bisher nicht offenbart.

Aufgabe der Erfindung war die Herstellung eines gegenüber dem Stand der Technik geruchsintensiveren Duftstoffes der Holz- und Ambraklasse, der sich außerdem durch verbesserte Eigenschaften wie Haftfähigkeit, Geruchsschwellenwert und eine niedrige Verdünnungsgrenze auszeichnet.

Erfindungsgemäß wurde die Aufgabe gelöst durch (-)-2-Cyclododecylpropanol.

Überraschenderweise wurde auch gefunden, daß während sich das (-)-Enantiomer hervorragend als Duftstoff eignet, sich das fast geruchslose (+)-Enantiomer vorteilhaft als Fixateur für andere Duftstoffe eignet. Es sorgt für eine langanhaltende Haftung, ohne den Eigenduft des anhaftenden Duftstoffes durch einen starken Eigengeruch in unerwünschter Weise zu beeinträchtigen.

Die Herstellung der beiden Enantiomeren von 2-Cyclododecylpropionsäure, der Vorstufe bzw. dem Zwischenprodukt zur Herstellung von Cyclododecylpropanol gelingt durch Racematspaltung von bekannter racemischer 2-Cyclododecylpropionsäure durch Kristallisation der diastereomeren Salze mit einem optisch aktiven Amin als Hilfsmittel und anschließende Freisetzung des jeweiligen Enantiomeren der Säure aus dem Salz, z.B. durch Umsetzung mit einem stark sauren Ionentauscher. Zur weiteren Reinigung kann die Kristallisation des diastereomeren Salzes und die anschließende Freisetzung der Säure wiederholt durchgeführt werden. Außerdem kann die enantiomerenreine Säure durch Umkristallisieren weiter gereinigt werden (Houben-Weyl, Methoden der Organischen Chemie, Band IV, Allgemeine chemische Methoden, Teil 2, Georg Thieme Verlag Stuttgart, 1955, S. 505-532).

Die beiden Enantiomeren des 2-Cyclododecylpropanols werden durch Reduktion der Säurefunktion der entsprechenden Enantiomeren der 2-Cyclododecylpropionsäure hergestellt. Die Reduktion kann z.B. durch katalytische Hydrierung oder mit einem komplexen Hydrid, wie z.B. Lithiumaluminiumhydrid erfolgen.

Gegenstand der Erfindung ist daher auch die Verwendung von (+)-bzw. (-)-2-Cyclododecylpropionsäure als Zwischenprodukt zur Herstellung von (-)- bzw. (+)-2-Cyclododecylpropanol.

Überraschenderweise ist der Geruch der Enatiomeren des 2-Cyclododecylpropanols völlig unterschiedlich. Während das (+)-Enantiomere fast geruchlos ist, besitzt das (-)-Enantiomere einen wesentlich stärkeren Geruch als die racemische Mischung. Der Geruch von racemischem 2-Cyclododecylpropanol ist praktisch ausschließlich durch das (-)-Enantiomere bestimmt. Zwar ist es bekannt, dass sich Enantiomere im Geruch unterscheiden können (z.B. (+)-Carvon [Kümmel], (-)-Carvon [Krauseminze], L. Friedman, J.G. Miller, "Odor Incongruity and Chirality", Science 1971, 172, 1044, zitiert nach G. Ohloff "Riechstoffe und Geruchsinn", S.42, Springer-Verlag 1990). Gewöhnlich sind die Unterschiede qualitativer Natur und nur mehr oder weniger stark ausgeprägt. Die völlige Diskriminierung des Geruches der beiden Enantiomeren in stark riechend bzw. geruchlos ist äußerst selten (vergl. G. Ohloff "Riechstoffe und Geruchsinn", S.44, Springer-Verlag 1990) und bei einfachen Molekülen mit nur einem Asymmetriezentrum bisher nicht beschrieben.

Besonders vorteilhaft für die Anwendung in Parfümölen ist, daß das (-)-Enantiomer mit allen gängigen, auf dem Markt befindlichen Riechstoffen, wie z.B. der Klassen der cyclischen und acyclischen Terpene, der aliphatischen, cycloaliphatischen, aromatischen Riechstoffe, der Phenolderivate oder der Heterocyclen sehr gut verträglich ist.

### Beispiele

### Beispiel 1

### (-)-2-Cyclododecylpropionsäure

215 g (0,85 mol) racemische 2-Cyclododecylpropionsäure mit einer Reinheit von 95 % wurden bei 50 °C in 250 ml Toluol gelöst und anschließend 52,6 g (0,425 mol) (R)-(+)-1-Phenylethylamin zugegeben. Dann gab man ebenfalls bei 50 °C 430 ml n-Hexan zu und ließ die klare Lösung abkühlen. Dabei fiel das Salz aus. Nach drei Tagen Stehen bei Raumtemperatur wurde das ausgefallene Salz abgesaugt und mit n-Hexan gewaschen. Nach dem Trocknen erhielt man 85 g des Aminsalzes. Die Lösung dieses Salzes in 2,4 l Methanol ließ man dann durch eine Schüttung von 1 l stark saurem Ionentauscher (Dowex 50 W x 8) in einer Glassäule mit 500 mm Höhe und 55 mm Durchmesser laufen und spülte anschließend mit Methanol nach. Dann wurde das Methanol am Rotationsverdampfer abgezogen. Man erhielt 56 g eines Öls, welches langsam zu kristallisieren begann. Der Rückstand wurde in 46 g warmem Aceton gelöst. Beim Abkühlen kristallisierten 16 g der Säure (nach Waschen mit kaltem Aceton und Trocknen) mit einem Gehalt nach GC an einer chiralen Säule von 62 % (-)- und 38 % (+)-2-Cyclododecylpropionsäure. Das Filtrat ergab nach dem Einengen 42 g eines farblosen Öls mit einem Gehalt nach GC an einer chiralen Säule von 95 % (-)-und 5 % (+)-2-Cyclododecylpropionsäure. Dieses Öl wurde in 246 ml Toluol bei 50 °C gelöst und mit 18,9 g (R)-(+)-1-Phenylethylamin versetzt. Nach dem Abkühlen kristallisierten 47 g (0,13 Mol) des Aminsalzes aus, welches abgesaugt, mit n-Hexan gewaschen und an der Luft getrocknet wurde. Das getrocknete Aminsalz wurde in 1,3 1 Methanol gelöst, wieder über Ionentauscher vom Amin befreit und die Lösung dann am Rotationsverdampfer eingeengt. Man erhielt 30 g eines farblosen Öls, welches langsam kristallisierte. Der über GC an einer chiralen Säule bestimmte Gehalt an (-)-2-Cyclododecylpropionsäure lag bei > 98 %.

### Beispiel 2

### (+)-2-Cyclododecylpropionsäure

Das Filtrat der 1. Kristallisation des Aminsalzes aus Beispiel 1 wurde am Rotationsverdampfer eingedampft. Man erhielt 222 g eines Öls, welches in 2200 ml Methanol gelöst und filtriert wurde. Die filtrierte Lösung ließ man dann durch eine Schüttung von 1 l stark sauren Ionentauscher (Dowex 50 W x 8) in einer Glassäule mit 500 mm Höhe und 55 mm Durchmesser laufen und spülte anschließend mit Methanol nach. Dann wurde das Methanol am Rotationsverdampfer abgezogen. Man erhielt 128 g eines Öls, welches langsam zu kristallisieren beginnt. Dieses Öl wurde in 752 ml Toluol bei 50 °C gelöst und mit 42,2 g (S)-(-)-1-Phenylethylamin versetzt. Nach dem Abkühlen kristallisierten 92,5 g des Aminsalzes aus, welches abgesaugt, mit n-Hexan gewaschen und an der Luft getrocknet wurde. Das getrocknete Aminsalz wurde in 2,5 l Methanol gelöst, wieder über Ionentauscher vom Amin befreit und die Lösung dann am Rotationsverdampfer eingeengt. Man erhielt 55,5 g eines farblosen Öls mit einem Gehalt (GC an einer chiralen Säule) von 90 % (-)-2-Cyclododecylpropionsäure und 10 % (+)-2-Cyclododecylpropionsäure.

Dieses Öl wurde in 500 ml Toluol bei 60 °C gelöst und mit 24,5 g (S)-(-)-1-Phenylethylamin versetzt. Nach dem Abkühlen kristallisierten 65 g des Aminsalzes aus, welches abgesaugt, mit n-Hexan gewaschen und an der Luft getrocknet wurde. Das getrocknete Aminsalz wurde in 1,8 l Methanol gelöst, wieder über Ionentauscher vom Amin befreit und die Lösung dann am Rotationsverdampfer eingeengt. Man erhielt 43 g eines farblosen Öls mit einem Gehalt (GC an einer chiralen Säule) von > 97 % (+)-2-Cyclododecylpropionsäure.

### Beispiel 3

### (+)-2-Cyclododecylpropanol

7 g Lithiumaluminiumhydrid (0,183 mol) wurden in 400 ml wasserfreiem THF vorgelegt und unter Stickstoffatmosphäre bei schwachem Rückfluss unter Rühren 30 g (0,125 mol) (-)-2-Cyclododecylpropionsäure aus Beispiel 1 in 100 ml wasserfreiem THF innerhalb von 3 h zugetropft. Dann wurde noch 4 h am Rückfluss gekocht. Anschließend wurden bei Raumtemp. 12 ml 10 %ige Kaliumhydroxid-Lösung zugetropft und die Mischung noch 15 min. am Rückfluß gekocht. Die ausgefallenen Aluminiumsalze wurden abfiltriert und mit THF gewaschen und dann noch zwei mal mit je 250 ml THF ausgekocht. Die gesammelten THF-Filtrate wurden am Rotationsverdampfer eingeengt. Der Rückstand wurde in 150 ml Toluol aufgenommen, mit gesättigter Natriumchlorid-Lösung gewaschen und über Natriumsulfat getrocknet. Schließlich wurde das Toluol am Rotationsverdampfer abgezogen und der verbleibende Rückstand destilliert. Man erhielt 24,2 g eines farblosen Öls mit einer Reinheit von > 97 % (+)-2-Cyclododecylpropanol. Der Drehwert von 250 mg dieser Verbindung in 10 ml abs. Ethanol betrug +26,0 Grad.

Geruchliche Charakterisierung: sehr schwach, fast geruchlos

### Beispiel 4

### (-)-2-Cyclododecylpropanol

10 g Lithiumaluminiumhydrid (0,264 mol) wurden in 400 ml wasserfreiem THF vorgelegt und unter Stickstoffatmosphäre bei schwachem Rückfluss unter Rühren 43 g (0,179 mol) (+)-2-Cyclododecylpropionsäure aus Beispiel 2 in 130 ml wasserfreiem THF innerhalb von 3 h zugetropft. Dann wurde noch 4 h am Rückfluss gekocht. Anschließend wurden bei Raumtemp. 12 ml 10 %ige Kaliumhydroxid-Lösung zugetropft und die Mischung noch 15 min. am Rückfluß gekocht. Die ausgefallenen Aluminiumsalze wurden abfiltriert und mit THF gewaschen und dann noch zwei mal mit je 250 ml THF ausgekocht. Die gesammelten THF-Filtrate wurden am Rotationsverdampfer eingeengt. Der Rückstand wurde in 150 ml Toluol aufgenommen, mit gesättigter Natriumchlorid-Lösung gewaschen und über Natriumsulfat getrocknet. Schließlich wurde das Toluol am Rotationsverdampfer abgezogen und der verbleibende Rückstand destilliert. Man erhielt 32 g eines farblosen Öls mit einer Reinheit von > 97 % (-)-2-Cyclododecylpropanol. Der Drehwert von 250 mg dieser Verbindung in 10 ml abs. Ethanol betrug -25,2 Grad.

Geruchliche Charakterisierung:
Geruchscharakter: stark ambraartig-holzig, mit dominierendem ambra Charakter und linearem Duftverlauf
Intensität: sehr stark, wesentlich stärker als racemisches Produkt
Dufthaftung
   (10%ige Lösung in Ethanol): > 1 Monate
(+)-Enantiomer:
   holzig-linear, keine signifikanten Duftfacetten, Haftung größer 1 Monat

## Patentansprüche

1. (+)-2-Cyclododecylpropanol sowie dessen Derivate und Ester.

2. (-)-2-Cyclododecylpropanol sowie dessen Derivate und Ester.

3. (+)-2-Cyclododecylpropionsäure sowie deren Salze.

4. (-)-2-Cyclododecylpropionsäure sowie deren Salze.

5. Verwendung der Verbindung gemäß Anspruch 1 oder 2 als Komponente in Parfümölmischungen oder als Fixateur.

6. Verwendung der Verbindung gemäß Anspruch 2 als Duftstoff.

7. Verfahren zur Herstellung von (-)-2-Cyclododecylpropanol, **dadurch gekennzeichnet, daß** man (+)-2-Cyclododecylpropionsäure reduziert.

8. Verfahren zur Herstellung von (+)-2-Cyclododecylpropanol, **dadurch gekennzeichnet, daß** man (-)-2-Cyclododecylpropionsäure reduziert.

9. Verwendung von (+)-2-Cyclododecylpropionsäure gemäß Anspruch 2, als Zwischenprodukt zur Herstellung von (-)-2-Cyclododecylpropanol.

10. Verwendung von (-)-2-Cyclododecylpropionsäure gemäß Anspruch 2, als Zwischenprodukt zur Herstellung von (+)-2-Cyclododecylpropanol.

## Claims

1. (+)-2-Cyclododecylpropanol or a derivative or ester thereof.

2. (-)-2-Cyclododecylpropanol or a derivative or ester thereof.

3. (+)-2-Cyclododecylpropionic acid or a salt thereof.

4. (-)-2-Cyclododecylpropionic acid or a salt thereof.

5. The use of the compound as claimed in claim 1 or 2 as a component in perfume oil mixtures or as a fixative.

6. The use of the compound as claimed in claim 2 as a fragrance.

7. A process for the preparation of (-)-2-cyclododecylpropanol, which comprises reducing (+)-2-cyclododecylpropionic acid.

8. A process for the preparation of (+)-2-cyclododecylpropanol, which comprises reducing (-)-2-cyclododecylpropionic acid.

9. The use of (+)-2-cyclododecylpropionic acid as claimed in claim 2 as an intermediate for the preparation of (-)-2-cyclododecylpropanol.

10. The use of (-)-2-cyclododecylpropionic acid as claimed in claim 2, as an intermediate for the preparation of (+)-2-cyclododecylpropanol.

## Revendications

1. Le (+)-2-cyclododécylpropanol, ses dérivés et esters.

2. Le (-)-2-cyclododécylpropanol, ses dérivés et esters.

3. L'acide (+)-2-cyclododécylpropionique et ses sels.

4. L'acide (-)-2-cyclododécylpropionique et ses sels.

5. Utilisation du composé selon la revendication 1 ou 2 en tant que composant de compositions de parfums ou en tant que fixateur.

6. Utilisation du composé selon la revendication 2 en tant que matière odorante.

7. Procédé pour la préparation du (-)-2-cyclododécylpropanol, **caractérisé par le fait que** l'on réduit l'acide (+)-2-cyclododécylpropionique.

8. Procédé de préparation du (+)-2-cyclododécylpropanol, **caractérisé par le fait que** l'on réduit l'acide (-)-2-cyclododécylpropionique.

9. Utilisation de l'acide (+)-2-cyclododécylpropionique selon la revendication 2 en tant que produit intermédiaire de la préparation du (-)-2-cyclododécylpropanol.

10. Utilisation de l'acide (-)-2-cyclododécylpropionique selon la revendication 2 en tant que produit intermédiaire de la préparation du (+)-2-cyclododécylpropanol.
